# EUROPEAN PATENT SPECIFICATION

(11) **EP 1 825 819 B1**
(45) Date of publication and mention of the grant of the patent: **01.04.2009**
(21) Application number: 07250740.3
(22) Date of filing: 21.02.2007
(51) Int. Cl.: A61B 17/076, A61B 17/04

(54) **Devices for fastener removal**
Vorrichtungen zur Entfernung von Befestigungen
Dispositifs de suppression de fixation

(30) Priority: 22.02.2006 US 360033
(43) Date of publication of application: 29.08.2007
(73) Proprietor: ETHICON ENDO-SURGERY, INC., Cincinnati, OH 45242-2839 (US)
(72) Inventor: Ortiz, Mark S., Milford, Ohio 45150 (US)
(74) Representative: Tunstall, Christopher Stephen

(56) References cited:
- US-A- 5 451 231
- US-A- 5 562 681
- US-A- 5 725 554

## Description

### FIELD OF THE INVENTION

The present invention is directed broadly toward devices for removing fasteners, especially fasteners used to bind tissue in various patient treatment regimes.

### BACKGROUND OF THE INVENTION

Severe obesity is a major health risk that can decrease life expectancy and give rise to a number of other associated ailments including the onset of cardiovascular disease, hypertension, diabetes and severe arthritis. A number of surgical procedures can be performed to aid in the treatment of obesity. One example is a gastric restriction in which one or more fasteners are inserted into gastric tissue to hold the tissue in a folded configuration that effectively reduces the effective volume of a patient's stomach.

Removal of the fasteners may be desirable in a number of circumstances. For example, it may be desirable to perform a temporary gastric restriction and subsequently to perform a more permanent procedure such as a Roux-en-y at a chosen time. Removal of the fasteners is typically required before such a permanent procedure can be performed. In another instance, a patient's difficulty in tolerating the consequences associated with having a gastric restriction can also drive a desire to have gastric fasteners removed before they typically pass through a patient's system. When metal fasteners are utilized to bind the gastric tissue, such fasteners can be difficult to remove due to their inherent strength. As well, the labor and risks typically associated with open surgical procedures can present further complications when removal of implanted fasteners is desired. United States patent no. 5562681 provides a staple remover for removing surgical staples having side members spaced from each other in a fired position at a staple spacing.X

Accordingly, a need exists for devices and techniques that can be utilized to remove tissue fasteners in a convenient fashion.

### SUMMARY OF THE INVENTION

In one exemplary embodiment, a surgical device for removing fasteners includes an elongate body with a distally extending fastener catch for selectively engaging tissue-attached fasteners. Elongate bodies can be flexible and can be disposed within a device such as an endoscope, for example, to allow their delivery. Alternatively or in addition, a substantially hollow delivery conduit can used to house the elongate body, while allowing the body to be advanceable through the conduit. An elongate body can also include one or more working channels to allow endoscopic/laparoscopic tools, such as an optical device adapted for visualization, to be disposed therein. The fastener catch of an elongate body can be rigidly attached, or can be movable relative to the body. As well, the fastener catch can include a structure that is adapted to selectively engage a loop fastener. The device can also include a fastener-severing member for severing fasteners, which can be optionally movable with respect to the fastener catch. The fastener-severing member can be a tubular structured, energy-delivery member, such as a portion of an Argon Plasma Coagulator. Accordingly, the fastener catch can include a structure adapted shield tissue from contact with fastener-severing energy. At least one fastener-removal element can also be included with the device, which can also be moveable with respect to the fastener catch. The fastener-removal element can be disposed within a channel of the elongate body, and can optionally include a forceps. In one instance, two or more fastener-removal elements can be utilized with the device. In general, one or more portions of the device can be reconditioned after at least one use of the device. Such reconditioning can include replacing or cleaning at least a portion of any one of the pieces of the device, as well as optionally disassembling or reassembling the device.

### BRIEF DESCRIPTION OF THE DRAWINGS

The invention will be more fully understood from the following detailed description taken in conjunction with the accompanying drawings, in which:

FIG. 1 presents a perspective, partial-cut-away view of a device for extracting fasteners consistent with an embodiment of the invention;

FIG. 2 presents a perspective view of the distal end of a fastener extracting device that is engaging a looped fastener;

FIG. 3 presents a perspective view of the distal end of the device shown in FIG. 2 having forceps that are grasping the looped fastener, the fastener-severing member in an advanced position, and an optical probe advanced from the elongate body;

FIG. 4 presents a perspective view of the proximal end of the device shown in FIG. 1;

FIG. 5A presents a perspective view of an elongate body having a rigidly attached fastener catch;

FIG. 5B presents a close-up perspective view of a portion of an elongate body with a movable fastener catch coupled thereto; and

FIG. 6 presents a perspective view of the distal end of a fastener catch engaging a looped fastener, which penetrates tissue.

### DETAILED DESCRIPTION OF THE INVENTION

Certain exemplary embodiments will now be described to provide an overall understanding of the principles, structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with features of other embodiments. Such modifications and variations are intended to be included within the scope of the present invention. For example, any feature described herein regarding any one of a fastener-catch, a fastener-severing member, or a fastener removal element can be combined with another feature to provide an embodiment within the scope of the present invention.

Methods of surgically reversing a gastric restriction created using one or more fastener elements that penetrate gastric tissue are encompassed by several embodiments. In general, fastener elements can be disengaged from gastric tissue and removed from the tissue-attachment site to effect reversal of the restriction. A fastener-extraction device for disengaging and/or removing fastener elements can be delivered to the gastric restriction site using an endoscopic or laparoscopic technique. For example, the device can be delivered trans-orally, through the esophagus, to the stomach. The fastener-extraction device can then be positioned in proximity to an attached fastener element, which can allow the device to disengage and/or remove the fastener element. Fastener-extraction devices utilized with the method can be adapted to disengage and/or remove the type of fastener element utilized in the gastric restriction. Exemplary types of fastener elements include sutures, clips, staples, looped fasteners, and other fasteners that can be constructed from a variety of suitable materials. Disengagement of a fastener element can be achieved by means of a mechanically-based mechanism such as engaging a fastener element and pulling the fastener from the tissue. This technique can be useful when a fastener element is attached to the stomach wall and the stomach is inflated before the fastener element is pulled out of the wall. In cases where the fastener is tightly bound to tissue such that pulling the element from the tissue can result in a tissue tear, other disengagement mechanisms may be desirable. Accordingly, disengagement of a fastener element can also be effected by other mechanical-based mechanisms such as deforming and severing the fastener element. In one example, when staples are used to effect a gastric restriction, a surgical staple remover can be employed to extract the staples by staple deformation and/or cutting. In another example, when sutures are utilized as fasteners, endoscopic scissors can be used to sever the sutures. In some instances, particularly when fasteners are constructed of plastics or metals, severing the fastener element can be achieved by applying energy to the fastener element. For example, an Argon Plasma Coagulator can be used to effect severing of a looped fastener made of a metal such as nitinol. In several instances, embodiments of devices described herein can be effectively utilized as fastener-extraction devices to effect reversal of a gastric restriction, though a variety of other fastener-extraction devices can also be utilized.

One exemplary embodiment of a surgical device for removing fasteners includes a substantially elongate body having a fastener catch that can extend distally from the elongate body. The fastener catch can be adapted to selectively engage a fastener that is attached to tissue such as gastric tissue. A fastener-severing member can be included that can be capable of moving with respect to the fastener catch. The device can also include one or more fastener removal elements that are optionally adapted to move with respect to the fastener catch. Such a device can be used to excise or sever fasteners that are attached to tissue. Furthermore, the device can be adapted to be delivered using an endoscopic or laparoscopic technique. Endoscopic and laparoscopic surgical instruments are often preferred over traditional open surgical devices since the use of natural orifices (endoscopic) or smaller incisions (laparoscopic) tends to reduce the post-operative recovery time and complications. Accordingly, in such a configuration, the device can be employed to remove fasteners in a manner consistent with the employment of minimally-invasive surgical techniques.

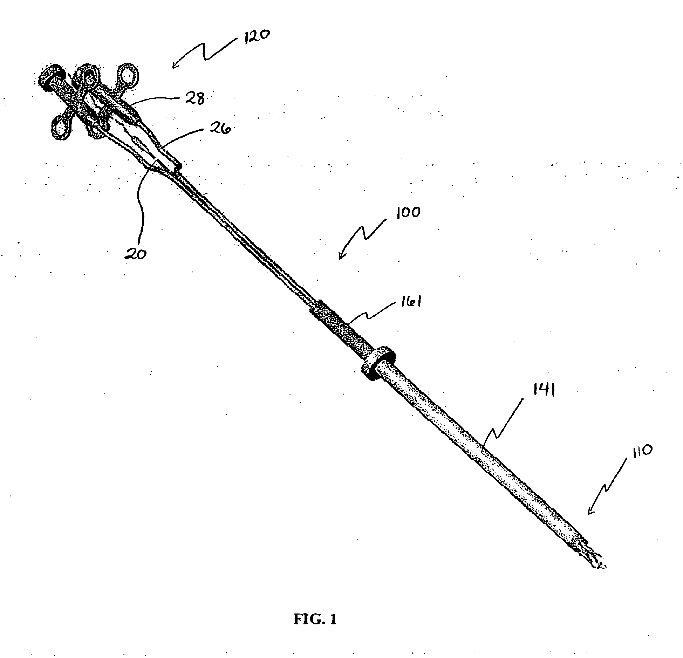

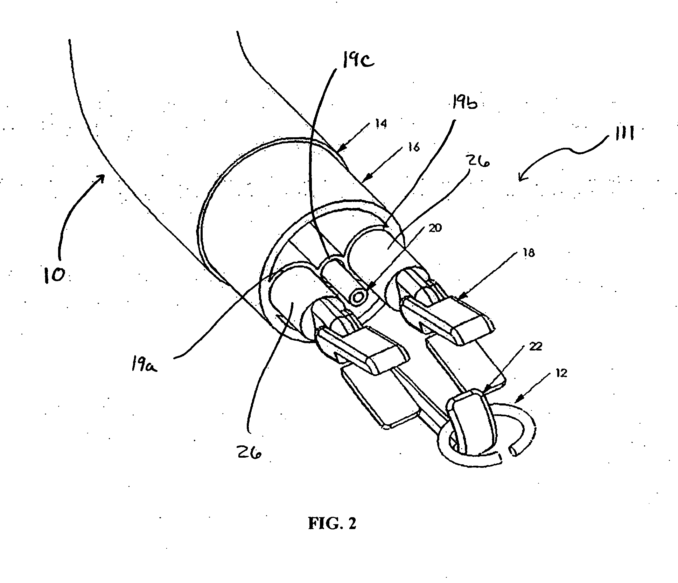

FIGS. 1 and 2 provide illustrations of an embodiment of a device for extracting fasteners. The overall view depicted in FIG. 1 shows the distal end 110 of the device 100 emerging from a hollow delivery conduit 141. The conduit 141 can surround at least a portion of an elongate body 161 that can act as a housing for elements 20, 26, which are controlled at the proximal end 120 of the device 100. As depicted in FIG. 1, the conduit 141 is partially cutaway toward the proximal end to reveal the elongate body 161. The length between the distal end and the proximal end of the device can vary, and can be chosen to suit the requirements of a surgical procedure for which the device 100 is to be utilized. For example, a length of approximately three feet can be appropriate when the device is trans-orally delivered to a gastric site, while a length of about six feet can allow the distal end to reach bowel sites. In addition, the length of the device between the distal end and the proximal end can be disposed to have flexibility in directions substantially perpendicular to the axis of the elongate body. Such flexibility can advantageously allow the device to be manipulated through a tortuous path in a patient's body. The device can entirely flexible, or it can have segments and/or regions that possess more flexibility relative to other parts of the device.

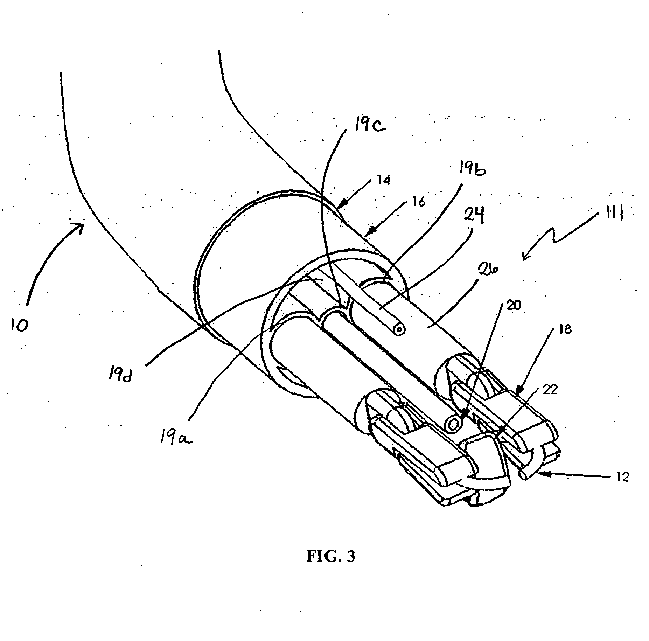

FIG. 2 provides an illustration of a distal end 111 of a fastener-extraction device according to one embodiment. The elongate body 16 can be slidably movable relative to a hollow delivery conduit 14 that can have a generally tubular shape. The body 16 can include a fastener catch 22 that extends distally from the body 16. The fastener catch 22, as depicted in FIG. 2, can have a hook-like shape adapted to selectively engage a fastener 12. The body 16 can also include channels 19a, 19b, 19c for housing a fastener-severing member and two fastener-removal elements 26. In one embodiment, each of the fastener-severing member and fastener-removal elements 26 can be movable with respect to the fastener catch 22. The fastener-severing member shown in FIG. 2 can include a tubular structure 20 that is effective for delivering energy to sever a fastener 12. Each of the fastener-removal elements 26 can include grasping jaws such as forceps 18 that can grasp a section of a fastener 12. As shown in FIGS. 2 and 3, the distal end 111 can be configured to have a slight curvature 10 to enhance the device's ability to be positioned and properly oriented within an internal region of a patient. In such an instance, the portion of the device distal to the curvature 10 can be stiffer than the section proximal to the curvature 10 to maintain the preferred curvature. Such a curvature can predispose the distal end of the device to be naturally positioned and oriented toward the gastric wall upon trans-oral insertion into a patient.

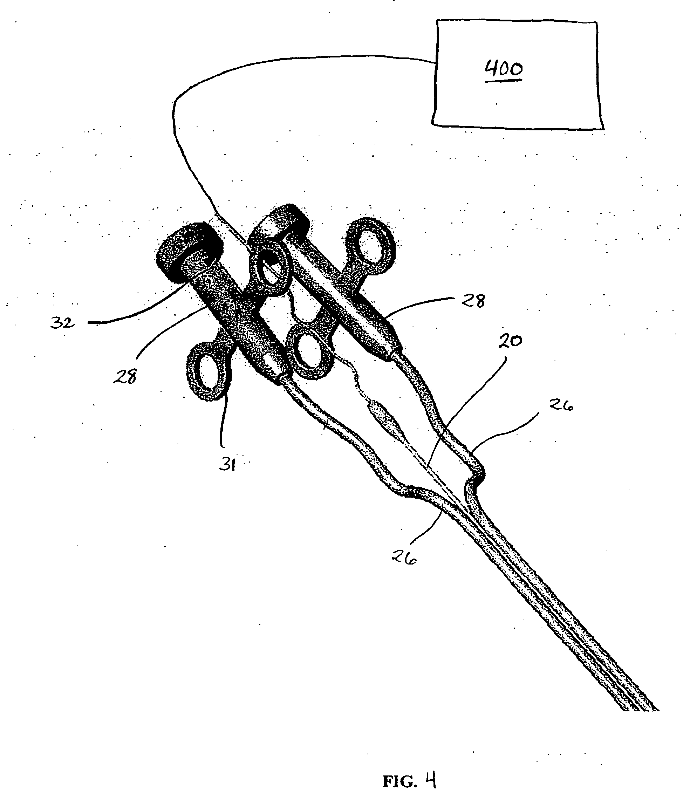

Control of the various portions of the distal end 110 of the fastener-extraction device 100 can be effected by manipulating the proximal end 120 of the device 100 as depicted in FIGS. 1 and 4. The proximal end of the elongate body 161, shown in FIG. 1, can be advanced and retracted relative to the hollow delivery conduit 141 to help position portions of the device, such as the fastener catch. As shown in FIG. 4, fastener-removal elements 26 can be advanced and retracted relative to the elongate body using handles 28 that are coupled to the distal end of the removal elements 26. Actuators 31 can slide within slots 32 of handles 28 to actuate movement of the forceps at the distal end of the removal elements 26. The proximal end of the fastener-severing member 20 can also be moved to allow advancement and retraction of the member 20 relative to the fastener catch. When the member 20 is an energy delivering member, the proximal end of the member 20 can be coupled to a device 400 for delivering the energy (e.g., an Argon Plasma Coagulator). Those skilled in the art will readily appreciate that a variety of other mechanisms and arrangements can be utilized to control the various portions of fastener-extraction devices as described herein. For example, a housing can be used to conveniently couple the various pieces of the proximal end together. As well, various types of switches, sliders, buttons, triggers, knobs, levers, or other actuators can be used to effect closure of forceps or advancement and retraction of fastener-severing members and fastener-removal elements. All such variations are encompassed within the scope of the present application.

The hollow delivery conduit need not be a tubular structure as shown in FIG. 1, but can utilize any geometry that is effective to allow proper functioning and delivery of an elongate body and/or other portions of a fastener-extraction device. For example, when utilized in minimally invasive surgical techniques, the hollow delivery conduit can be a housing or a portion of an endoscope or a laparoscope. Alternatively, the delivery conduit can be independent of an endoscope or a laparoscope. The conduit can act to protect tissue from excessive frictional contact with the elongate body as the body is repeatedly inserted and retracted to remove individual fasteners or pieces thereof. The elongate body need not fill the cross-sectional cavity of the conduit as shown in FIGS. 2 and 3, but can fill a portion of the cross-sectional area of the cavity. Alternatively, the hollow delivery conduit can itself be inserted within an endoscope or a laparoscope. One skilled in the art will appreciate that though advantages may be accrued from the use of a hollow delivery conduit, such a structure is not necessary for the functioning of the fastener-extraction device.

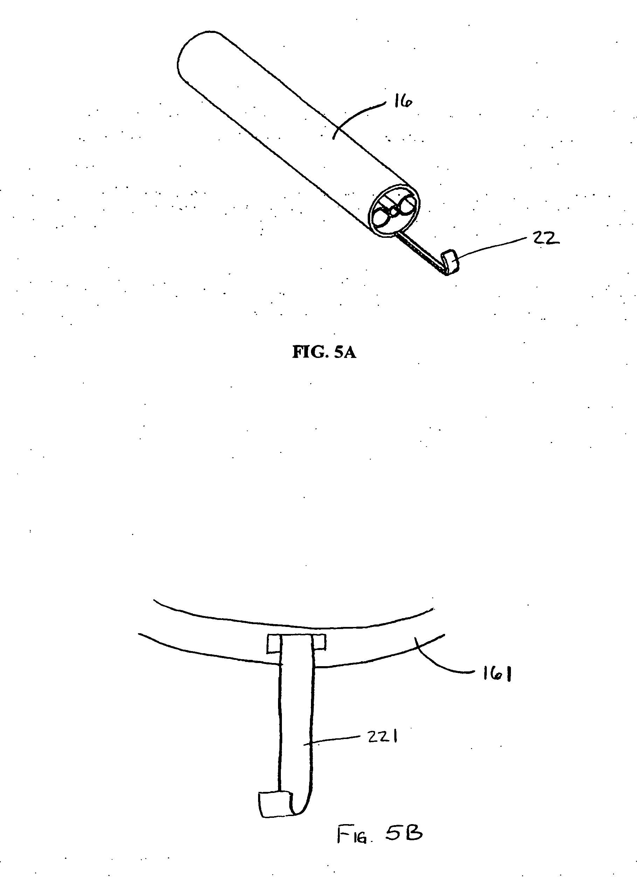

The elongate body of a fastener-extraction device can be arranged in a variety of suitable configurations effective to allow a distally positioned fastener catch to selectively engage a fastener attached to tissue. In one embodiment, as depicted in FIG. 5A, the fastener catch 22 can be rigidly attached to the remainder of the body 16. Accordingly, by moving the entire body 16, the fastener catch can be positioned as needed. Alternatively, the fastener catch 221 can be movable relative to the body 161, as depicted in FIG. 5B, to allow the catch 221 to advance and retract relative to the body 161. Also as depicted in FIGS. 2 and 3, the elongate body can have one or more separate channels (i.e., channels 19a, 19b, 19c) for guiding various portions of the device such as the fastener-severing member and the fastener removal elements. Other channels (e.g., a working channel 19d) can also be included to accommodate auxiliary structures, though the use of segregated channels is not a prerequisite of a fastener-excising device. Auxiliary structures that can be included with a fastener-excising device, and optionally delivered through working channel 19d, include devices that are typically delivered in conjunction with an endoscope or a laparoscope. For example, an optical device adapted to enable visualization of the surgical site by the surgeon and/or surgical team can be included with the device. As depicted in FIG. 3, an optical probe 24 is advanced through a working channel 19d of the elongate body 16 to allow visualization of a site having tissue bound by a fastener. Types of optical devices include imaging devices such as still-imaging cameras, moving picture devices, or active imaging devices, as well as radiation sources and other illuminating devices to aid visualization of a body cavity or internal site. Though the illustrated embodiments depict an elongate body that acts as a housing for a fastener-severing member, fastener-removal elements, or an optical device, it is understood that such a configuration is not a prerequisite for the elongate body. For example, the elongate body can be a simple solid elongate structure with a distally attached fastener catch, any remaining elements of the device being separate from the elongate structure and contained within a hollow delivery conduit.

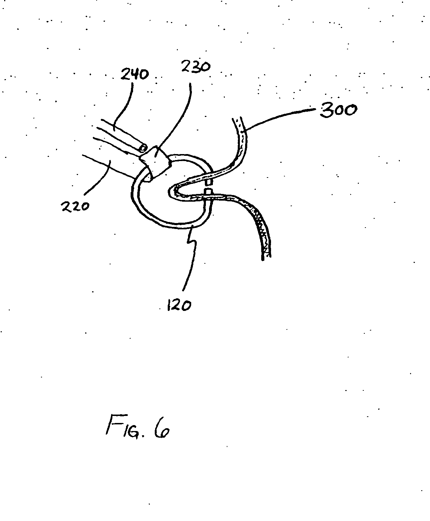

In general, fastener catches of an elongate body can have any configuration effective to engage a fastener. As depicted in FIGS. 2 and 3, the hook-like shape 22 of the fastener catch is effective to engage the loop fastener 12. Other geometries for a fastener catch, such as a L-shape or other shape adapted to engage a particular cross-sectional shape of a fastener, can also be effectively utilized. A fastener catch can also be adapted to shield tissue from direct contact by fastener-severing energy. As depicted in FIG. 6, a loop fastener 120 penetrates and binds a tissue layer 300. As the fastener catch 220 engages the fastener 120, a surface of the hook-like structure 230 facing away from tissue can effectively form a barrier between the tissue 300 and the engaged portion of the fastener 120. When energy-delivering member 240 is advanced adjacent to the looped fastener 120, and delivers energy that impinges on the fastener 120, the fastener catch can shield the tissue from direct energy exposure. Persons skilled in the art will appreciate that a variety of geometries can be utilized to configure fastener catches that can engage a fastener and/or shield tissue from energy contact beyond those explicitly disclosed herein.

Though the description of the fastener-extraction devices shown in FIGS. 2 and 3 provides some embodiments, fastener-severing members include a variety of devices that can be used to effectively sever a fastener. Appropriate fastener-severing members typically depend upon the properties of the fasteners utilized. Typical fasteners can include sutures, clips, staples, looped structures, and other geometries that can be formed from suitable materials. For example, when sutures are used as fasteners, scissors or other suture cutting devices can be employed as a portion of a fastener-severing member. When fasteners constructed with metal or other solids are utilized, mechanical cutting devices can be used, as well as fastener-severing members that deliver energy to the fastener. Sources of energy can be heat-based, electrically-based, radiation-based, sonically-based, or some combination thereof. As previously mentioned, an Argon Plasma Coagulation (herein "APC") device can be utilized to deliver an argon plasma to the portion of the fastener to be severed. In general, APC utilizes a source of argon gas and a high-frequency electrical generator. A catheter can be used to deliver the argon gas and electrical energy to a target region where the plasma is desired. Synchronization of argon gas delivery and a high-frequency electrical signal at an electrode results in gas ionization and formation of an argon plasma that is directed by the configuration of the distal end of the catheter. An argon plasma can be effectively used to sever fasteners constructed of metals such as nitinol. Of course, other devices and methods of producing other types of plasmas can also be effectively used by the embodiments described herein.

Fastener removal elements effective for use with the embodiments described herein can include devices capable of holding a fastener or a portion of a fastener that has been severed. Though the embodiment depicted in FIGS. 2 and 3 utilizes a pair of elements, each having a forceps disposed at the distal end of the element, other fastener-holding elements can be substituted. As well, the number of fastener-removal elements can also be varied. For example, one fastener removal element can be utilized even when a fastener is severed into multiple pieces. In such an instance, multiple insertions and retractions of the fastener-removal element can be performed to retrieve the numerous pieces of the severed fasteners. One skilled in the art will appreciate that the fastener-removal element can be constructed and configured such that they can be removed from the surgical site to extract fastener elements while the elongate body 16 remains at the surgical site. Alternatively, fastening elements can be removed as a result of the removal of the entire elongate body 16.

A fastener-extraction device configured to excise fasteners can be utilized to remove a gastric restriction performed on a patient's stomach. Fastener-excising devices, however, can find utility in other surgical applications that involve removal of fasteners (e.g., colonic applications). As such, a fastener-excising device can be used in a portion of a method to extract a tissue fastener that is attached to tissue. In one method, a fastener-excising device is delivered to an internal organ site of a patient having one or more tissue fasteners attached to tissue. As part of a minimally invasive surgical technique, such delivery can be accomplished endoscopically or laparoscopically. In such an instance, a delivery portal (e.g., any hollow delivery conduit or an endoscope/laparoscope having a working channel) can provide a pathway for transporting the remaining portions of the fastener-excising device. An optical device can also be delivered with the fastener-excising device to allow visualization at the internal organ site, and to aid guidance of the fastener-excising device. Such an optical device can be movably coupled to the fastener-excising device, or it can be an independently manipulated device.

Upon delivery to the internal organ site, the fastener-excising device can engage a tissue fastener and subsequently sever the fastener. As discussed earlier, severing of the tissue fastener can be effected by a variety of devices and methods. In one exemplary instance, energy is applied to the tissue fastener at a magnitude and for a duration effective to cause severing. Such energy can be applied in a variety of forms, such as a plasma. When energy is used to sever a fastener, tissue can be shielded from the energy to prevent limit tissue damage. For example, a fastener engager portion of the fastener-excising device can be positioned between the portion of the fastener to be severed and the tissue to be shielded from the severing energy.

After a tissue fastener is severed, the fastener can be removed from the internal organ site using one or more removal elements of the fastener-excising device. For example, plural removal elements can grasp a tissue fastener at locations on opposite sides of the site where a fastener is to be severed. After fastener severance, the pieces of the fastener can be removed by withdrawing the removal elements that each grasp a portion of the severed fastener. Such withdrawal can be effected by withdrawing the elongate body of the fastener-excising device when the body is coupled to each of the removal elements. The elongate body can then be reinserted into a delivery portal to effect further extraction of another tissue fastener at the internal organ site. This process can be repeated multiple times until all the fasteners have been removed. Skilled artisans will appreciate that other techniques for removing fasteners can also be employed. For example, one or more removal elements can be used to repeatedly remove the severed pieces of the tissue fastener, while the remainder of the device remains at the surgical site. In another example, removal elements can be employed to grasp pieces of a tissue fastener after the fastener is severed. All of these variations are clearly encompassed within the present application.

In one exemplary technique for operating a fastener-extraction device to remove fasteners, the elongate body 16 can be positioned within and advanced relative to the hollow delivery conduit 14 to engage a fastener 12 with fastener catch 22 as shown in FIG. 2. The fastener-removal elements 26 can then be advanced, and the forceps 18 actuated to grasp the fastener 12, as depicted in FIG. 3. The fastener-severing energy delivery member 20 can then be advanced to bring the distal end of the member 20 into close proximity with the fastener 12. A plasma, such as delivered by an Argon Plasma Coagulator, can be delivered through the member 20 and impinged upon the fastener to effect its severing. A surface of the fastener catch 22 can be positioned between the grasped fastener and bodily tissue to provide shielding of the tissue from direct plasma exposure. Subsequently, the elongate body 16 can be withdrawn through the conduit 14, the forceps 18 grasping pieces of the severed fastener 12, and thus effecting their removal from the site of the fastener implantation. The elongate body can be reinserted to the site of the next fastener to be removed, and the process can be repeated to remove all the fasteners. Those skilled in the art will appreciate the variations in using fastener-extraction devices, including those with different configurations than shown in FIGS. 2 and 3. In one example, one or more fastener-removal elements coupled to an elongate body can be used to remove one or more pieces of a severed fastener without removing all of the pieces during one withdrawal. Subsequent reinsertion of the removal-element(s) to the site allows one or more of the remaining pieces to be withdrawn. In another example, a fastener-extraction device can be configured to allow one or more fastener-removal elements to withdraw pieces of a severed fastener through an elongate body cavity or working channel without concurrent withdrawal of the body. Accordingly, withdrawal of the elongate body to effect withdrawal of a severed fastener is not required in such an instance.

Fastener-extracting devices, including portions thereof, can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. By way of example, the fastener-extracting device shown in FIGS. 1 and 2 can be reconditioned after the device has been used in a medical procedure. The device can be disassembled, and any number of the particular pieces (e.g., the hollow delivery conduit 141, the elongate body 161, the fastener-severing member 20, or the fastener-removal elements 20) can be selectively replaced or removed in any combination. For instance, the hollow delivery conduit can be replaced by a new conduit, while the remaining pieces are sterilized for reuse. Replacement of pieces can also include replacement of portions of particular elements, such as the replacement of a forceps on a distal end of a fastener-removal element. Upon cleaning and/or replacement of particular parts, the device can be reassembled for subsequent use either at a reconditioning facility, or by a surgical team immediately prior to a surgical procedure. Those skilled in the art will appreciate that reconditioning of a fastener-extracting device can utilize a variety of techniques for disassembly, cleaning/replacement, and reassembly. Use of such techniques, and the resulting reconditioned fastener-extracting device, are all within the scope of the present application.

Persons skilled in the art will understand that the devices and methods specifically described herein and illustrated in the accompanying drawings are non-limiting exemplary embodiments. The features illustrated or described in connection with one exemplary embodiment may be combined with the features of other embodiments. Such modifications and variations are intended to be included within the scope of the present invention. As well, one skilled in the art will appreciate further features and advantages of the invention based on the above-described embodiments. Accordingly, the invention is not to be limited by what has been particularly shown and described, except as indicated by the appended claims.

## Claims

1. A surgical device (100) for removing fasteners, comprising:
a substantially elongate body (16, 161) having a fastener catch (22) extending distally therefrom, the fastener catch (22) being adapted to selectively engage a fastener (12) attached to tissue; and
at least one fastener removal element (26),
**characterised in that** the surgical device (100) comprises a fastener-severing member (20) adapted to sever the fasteners.

2. The surgical device of claim 1, further comprising:
a substantially hollow delivery conduit (14, 141) adapted to house the elongate body such that the elongate body is advanceable therethrough.

3. The surgical device of claim 1, wherein the fastener-severing member (20) is an energy delivery member that includes a tubular structure.

4. The surgical device of claim 1, wherein the at least one fastener-removal element (26) is disposed within a channel (19a, 19b) of the elongate body.

5. The surgical device of claim 1, wherein the at least one fastener-removal element (26) includes a forceps (18).

6. The surgical device of claim 1, wherein the fastener catch (22) is rigidly attached to the elongate body.

7. The surgical device of claim 1, wherein the elongate body (16) is adapted to be disposed within an endoscope.

8. The surgical device of claim 1, wherein the elongate body includes at least one working channel (19d).

9. The surgical device of claim 8, further comprising an optical device (24) adapted for visualization disposed within the at least one working channel (19d).

10. The surgical device of claim 1, wherein at least a portion of the elongate body is flexible.

## Patentansprüche

1. Chirurgisches Gerät (100) zum Entfernen von Befestigungselementen, das aufweist:
einen im Wesentlichen länglichen Körper (16, 161) mit einem Fänger (22) für Befestigungselemente, der sich distal von ihm erstreckt, wobei der Fänger (22) für Befestigungselemente dazu ausgelegt ist, ausgewählt ein Befestigungselement (12) zu ergreifen, das an Gewebe befestigt ist;
wenigstens ein ein Befestigungselement entfernendes Element (26),
**dadurch gekennzeichnet, dass** das chirurgische Gerät (100) ein ein Befestigungselement abtrennendes Element (20) aufweist, das dazu ausgelegt ist, die Befestigungselemente abzuschneiden.

2. Chirurgisches Gerät nach Anspruch 1, das weiter aufweist:
eine im Wesentlichen hohle Zuführleitung (14, 141), die dazu ausgelegt ist, den länglichen Körper zu beherbergen, so dass der längliche Körper durch diese vorbewegbar ist.

3. Chirurgisches Gerät nach Anspruch 1, bei dem das ein Befestigungselement abtrennende Element (20) ein Energiezufuhrelement ist, das eine rohrförmige Struktur umfasst.

4. Chirurgisches Gerät nach Anspruch 1, bei dem das wenigstens eine ein Befestigungselement entfernende Element (26) innerhalb eines Kanals (19a, 19b) des länglichen Körpers angeordnet ist.

5. Chirurgisches Gerät nach Anspruch 1, bei dem das wenigstens eine ein Befestigungselement entfernende Element (26) eine Zange (18) umfasst.

6. Chirurgisches Gerät nach Anspruch 1, bei dem der Fänger (22) für Befestigungselemente starr an dem länglichen Körper befestigt ist.

7. Chirurgisches Gerät nach Anspruch 1, bei dem der längliche Körper (16) dazu ausgelegt ist, innerhalb eines Endoskops angeordnet zu werden.

8. Chirurgisches Gerät nach Anspruch 1, bei dem der längliche Körper wenigstens einen Arbeitskanal (19d) umfasst.

9. Chirurgisches Gerät nach Anspruch 8, weiter mit einer zur Visualisierung ausgelegten optischen Vorrichtung (24), die innerhalb des wenigstens einen Arbeitskanals (19d) angeordnet ist.

10. Chirurgisches Gerät nach Anspruch 1, bei dem wenigstens ein Teil des länglichen Körpers flexibel ist.

## Revendications

1. Dispositif chirurgical (100) pour retirer des fixations, comprenant :
■ un corps sensiblement allongé (16, 161) ayant un cliquet de fixation (22) s'étendant distalement de celui-ci, le cliquet de fixation (22) étant adapté pour mettre en prise sélectivement une fixation (12) fixée au tissu ;
■ au moins un élément de retrait de la fixation (26),
**caractérisé en ce que** le dispositif chirurgical (100) comprend un élément de séparation de la fixation (20) adapté pour séparer les fixations.

2. Dispositif chirurgical selon la revendication 1, comprenant en outre une conduite de distribution substantiellement creuse (14, 141) adaptée pour recevoir le corps allongé de telle sorte que le corps allongé puisse être avancé dans celle-ci.

3. Dispositif chirurgical selon la revendication 1, dans lequel l'élément de séparation de la fixation (20) est un élément de distribution d'énergie qui comprend une structure tubulaire.

4. Dispositif chirurgical selon la revendication 1, dans lequel au moins un élément de retrait de la fixation (26) est disposé dans un canal (19a, 19b) du corps allongé.

5. Dispositif chirurgical selon la revendication 1, dans lequel au moins un élément de retrait de la fixation (26) comprend une pince (18).

6. Dispositif chirurgical selon la revendication 1, dans lequel le cliquet de fixation (22) est fixé de façon rigide au corps allongé.

7. Dispositif chirurgical selon la revendication 1, dans lequel le corps allongé (16) est adapté pour être disposé dans un endoscope.

8. Dispositif chirurgical selon la revendication 1, dans lequel le corps allongé comprend au moins un canal de travail (19d).

9. Dispositif chirurgical selon la revendication 8, comprenant en outre un dispositif optique (24) adapté pour assurer la visualisation, disposé dans au moins un canal de travail (19d).

10. Dispositif chirurgical selon la revendication 1, dans lequel au moins une partie du corps allongé est flexible.
